# EUROPEAN PATENT APPLICATION

(11) **EP 1 418 182 A1**
(43) Date of publication of application: **12.05.2004**
(21) Application number: 02760561.7
(22) Date of filing: 07.08.2002
(51) Int. Cl.: C07K 14/47, C07K 1/16

(54) **METHOD OF PREPARING PEPTIDE FRAGMENT HAVING CELL DEATH INHIBITORY ACTIVITY**

(30) Priority: 09.08.2001 JP 2001242093
(71) Applicant: Juridical Foundation, The Chemo-Sero-Therapeutic Research Institute, Kumamoto-shi, Kumamoto 860-8568 (JP)
(72) Inventor: KAMEI, Shintaro, Kumamoto-shi, Kumamoto 862-0942 (JP); HAMAMOTO, Takayoshi, Kumamoto-shi, Kumamoto 860-0881 (JP); HIRASHIMA, Masaki, Kikuchi-gun, Kumamoto 861-1102 (JP); MAEDA, Hiroaki, Kumamoto-shi, Kumamoto 860-0076 (JP); TAKAHASHI, Kazuhiko, Sapporo-shi, Hokkaido 001-0037 (JP)
(74) Representative: VOSSIUS & PARTNER
(86) International application number: PCT/JP2002/008042
(87) International publication number: WO 2003/016347

(57) **Abstract**

A process for preparing a peptide fragment having a cell death-inhibitory activity is provided. In order to obtain a selenoprotein P fragment having a cell death-inhibitory activity, full-length selenoprotein P was reacted with various serine proteinases and the resulting fragments were electrophoresed and assessed for a cell death-inhibitory activity. A process for preparing a selenoprotein P fragment was established by identifying an enzyme that produced bands corresponding to a molecular weight of not more than 3.5 x 10⁴ in electrophoresis. A process for preparing a peptide fragment having a cell death-inhibitory activity according to the present invention may be used for amelioration, treatment and prevention of diseases caused by cell death and for more efficient production of useful living substances.

## Description

### TECHNICAL FIELD OF THE INVENTION

The present invention, belonging to the field of a medical drug and a reagent, relates to a process for preparing a protein having a novel function. Specifically, the present invention relates to a process for preparing, from a protein having a cell death-inhibitory activity or a fragment thereof, a peptide fragment or peptide fragments having more potent activity. More specifically, the present invention relates to a process for preparing a peptide fragments peptide fragments with a molecular weight of not more than 3.5 x 10⁴ having a potent cell death-inhibitory activity, using enzymatic hydrolysis for fragmentation. The process of the present invention may be used for preparing a large amount of a protein having a cell death-inhibitory activity and may effectively be applied for preparing a medicament for preventing worsening of, prophylaxis of, or treating various diseases, e.g. diseases associated with a cell death-inhibitory activity.

### BACKGROUND OF THE INVENTION

It has been suggested that cell death not only plays an important role in basic control of the nervous system, the endocrine system and the immune system in higher organisms but also is deeply involved in many diseases (Thompson C. B., Science, vol. 267, p.1456-1462 (1995). Some diseases including, for example, autoimmune diseases such as systemic lupus erythematosus, neurodegenerative diseases due to cell death of nerve cells, organ transplantation injuries associated with organ transplantation etc. may be regarded as one due to influence of cell death where apoptosis is involved.

While culturing cells, cell death is induced due to stress to cells imposed by substances from the cultured cells per se or from extraneous additives. However, it is not in all the cells that cell death is induced under certain conditions. For those cells that survived the circumstances, proteins necessary for suppressing the cell death-inducing signals due to stress under their thresholds should have already been expressed, or newly induced, either intracellularly or extracellularly. Such proteins include, as envisaged, transcription factor, synthases, enzymes related to metabolism, oxidoreductases, kinases, transferases, apoptosis-inhibiting proteins, etc. That is, sensitivity to stress in each of respective cells may vary due to difference in their expression level of these proteins. Thus, even if the mechanisms of cell death are not always the same, if the cell death-inducing signals could be suppressed under their thresholds by extraneously adding an inhibitory agent to cell death due to certain stress, then cell death could possibly be inhibited not only in cultured cells but also within the living body where similar stress occurred.

When certain culture cells are serum-free cultured, addition of a serum albumin fraction may induce cell death to the culture cells due to stress caused by a fatty acid contained in the serum albumin fraction. This cell death can be inhibited by adding human plasma. Recently, an active substance has been isolated and identified from human plasma. This substance is a carboxyl-terminal (hereinafter abbreviated as "C-terminal") fragment of a selenium-containing protein called selenoprotein P (Republished publication WO00/31131 (Japanese patent application No. 347863/1998)).

### DISCLOSURE OF THE INVENTION

Selenoprotein P in plasma is a glycoprotein with a molecular weight of about 6 x 10⁴ and is thought to contain ten selenocysteine residues. Selenoprotein P comprises mainly two domains, i.e. an N-terminal domain with one selenocysteine possessing an activity to reduce peroxide lipids, and a C-terminal domain with the rest of nine selenocysteines suggested to act as a selenium transmitter. Between these domains are two histidine rich regions, which are thought to mediate binding to anionic phospholipids or sulfated proteoglycans such as heparin on the cellular surface (Saito Y. et al., J. Health Science, vol. 46, p.409-413 (2000)). The selenoprotein P fragment obtained from human plasma as described above is a C-terminal peptide fragment that lacks the histidine rich regions.

For screening a cell death-inhibitory activity for isolation and identification of the C-terminal fragment of selenoprotein P as described above, a culture system of Dami cells, human megakaryoblasts, with serum free culture medium supplemented with albumin was suitably used. Dami cells may be subcultured on a mixed culture medium of an equal amount of RPMI 1640, D-MEM and F-12 supplemented with 0.1% BSA and 0.05 µM β-mercaptoethanol but can hardly grow on albumin-deprived medium. With culture medium containing 0.01 to 0.5% human serum albumin, the cells grow normally but on Day 4 and henceforth all the cells are put to death abruptly. A cell death-inhibitory activity in a sample may be measured by applying the sample to this culture system.

The isolated and identified fragment of selenoprotein P may also be used as an immunogen for eliciting a monoclonal antibody to thereby establish a simple method for purification of selenoprotein P using said monoclonal antibody, which method comprises providing a heparin-adsorbing fraction from normal human plasma, conducting ammonium sulfate fractionation and purification with affinity chromatography using said monoclonal antibody. Upon purification with this method, selenoprotein P was subject to fragmentation during the purification procedure, resulting in a series of proteins of bands corresponding to those with a molecular weight of not more than 3.5 x 10⁴ in SDS-PAGE under non-reductive condition. An amino-terminal (hereinafter abbreviated as "N-terminal") sequence analysis of the proteins in these bands revealed that these proteins contained both molecules starting from the 260th lysine residue and starting from the 292nd threonine residue.

Surprisingly, the C-terminal fragments of selenoprotein P had a higher cell death-inhibitory activity as compared to full-length selenoprotein P. Selenoprotein P was thought to subject to restricted degradation by a certain enzyme from human plasma during the purification procedure but said enzyme has not yet been identified. Besides, since the purified selenoprotein P fragments were obtained as several bands but not as a single molecule, a molecule possessing a high cell death-inhibitory activity still needs be identified from these bands. Moreover, the selenoprotein P fragments obtained by this method exhibited a varied ratio of each bands from preparation to preparation and hence poor reproducibility. As such, for enabling application of such selenoprotein P fragments with a potent cell death-inhibitory activity to a medicament, it has been desired to regulate a fragmentation process of selenoprotein P so that selenoprotein P fragments with a defined quality could stably and constantly be prepared.

As described above, selenoprotein P is subject to restricted degradation during the purification procedure. In order to investigate what enzyme is involved in this restricted degradation, the present inventors introduced various enzyme inhibitors into the purification process. As a result, the present inventors have found that fragmentation of selenoprotein P could entirely be inhibited with diisopropyl fluorophosphate (DFP), known to be a specific and potent inhibitor to a serine proteinase. Accordingly, the fragmentation may deeply involve a serine proteinase or some other protease that is secondarily activated by the serine proteinase.

Next, in order to search a desired enzyme, the present inventors aimed at serine proteinases in plasma, which were reacted with selenoprotein P to thereby search what enzyme restrictedly degraded selenoprotein P. One tenth amount of each of various enzymes at a weight ratio was added to the substrate selenoprotein P. After reaction for a fixed time, a portion of the reaction was treated with an SDS-PAGE solution and electrophoresed under non-reductive condition. Whether selenoprotein P was subject to restricted degradation was investigated with Coomassie Brilliant Blue dye. Serine proteinases tested include plasma kallikrein, factor XIIa, factor XIa, factor Xa, factor IXa, factor VIIa, thrombin, plasmin, tissue plasminogen activator, urokinase, trypsin and neutrophil elastase.

As a result, bands corresponding to a molecular weight of not more than 3.5 x 10⁴ were observed after treatment with most of the tested serine proteinases though an extent of degradation varied. This may suggest that a site sensitive to a serine proteinase is present anywhere in selenoprotein P.

Among the serine proteinases described above, it was found that plasma kallikrein, factor XIIa, factor XIa and plasmin most effectively degraded selenoprotein P. Conditions for enzymatic reaction of these enzymes may be conventional enzymatic conditions as usually employed in the art. It is both possible to regulate a reaction time by adjusting an amount of an enzyme to be added and alternatively to regulate an amount of an enzyme by adjusting a reaction time. In one preferable embodiment, not more than 1/10 amount, e.g. about 1/20 amount, at a weight ratio of an enzyme is added to the substrate selenoprotein P and the mixture is incubated at 37°C for 15 minutes to 24 hours to thereby produce desired selenoprotein P fragments.

The thus obtained reaction products exhibited a higher cell death-inhibitory activity than that observed before degradation, proving that desired selenoprotein P fragments were produced.

### BRIEF DESCRIPTION OF DRAWINGS

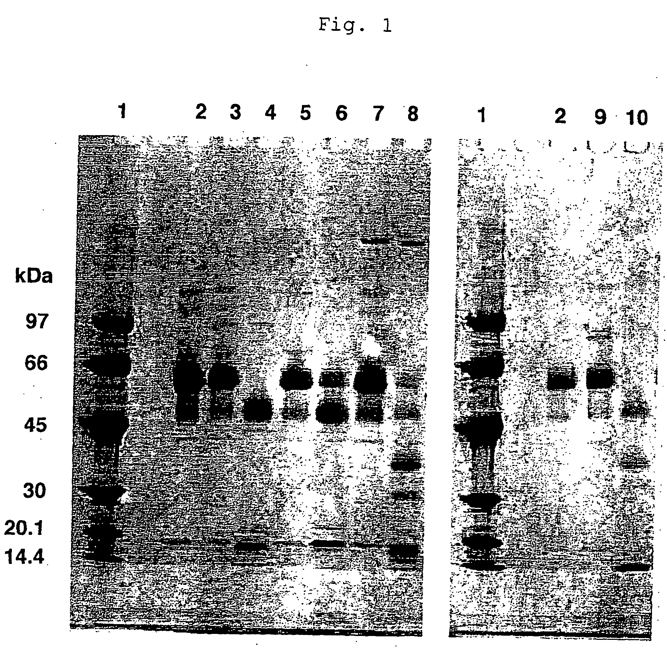

Fig. 1 shows results of SDS-PAGE electrophoresis of samples after restricted degradation of selenoprotein P with various serine proteinases (as an exemplary). Lane 1: MW marker; Lane 2: selenoprotein P; Lane 3: selenoprotein P plus plasma kallikrein (0 hour); Lane 4: selenoprotein P plus plasma kallikrein (24 hours); Lane 5: selenoprotein P plus factor XIIa (0 hour); Lane 6: selenoprotein P plus factor XIIa (24 hours); Lane 7: selenoprotein P plus factor XIa (0 hour); Lane 8: selenoprotein P plus factor XIa (24 hours); Lane 9: selenoprotein P plus plasmin (0 hour); Lane 10: selenoprotein P plus plasmin (24 hours).

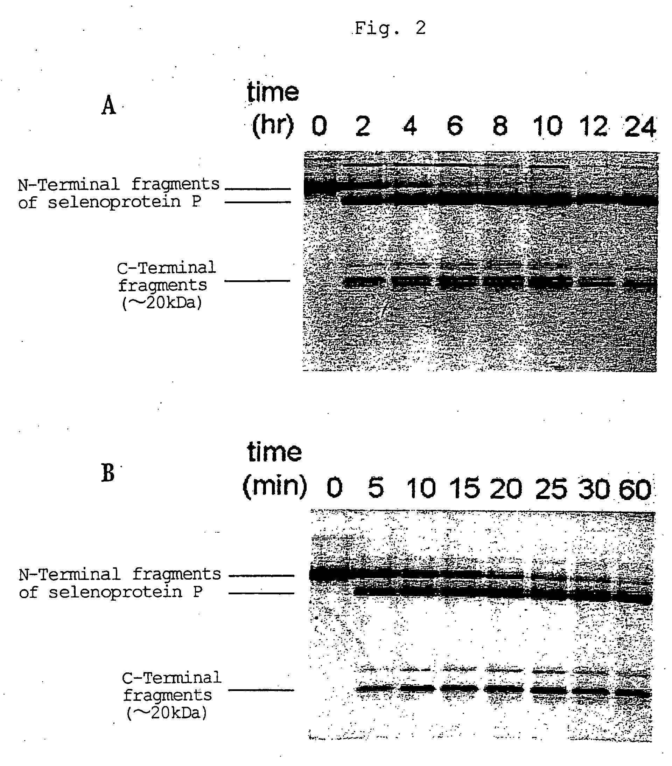

Fig. 2 shows results of SDS-PAGE electrophoresis of samples after restricted degradation of selenoprotein P with plasma kallikrein or plasmin. A: restricted degradation of selenoprotein P with plasma kallikrein in course of time; B: restricted degradation of selenoprotein P with plasmin in course of time.

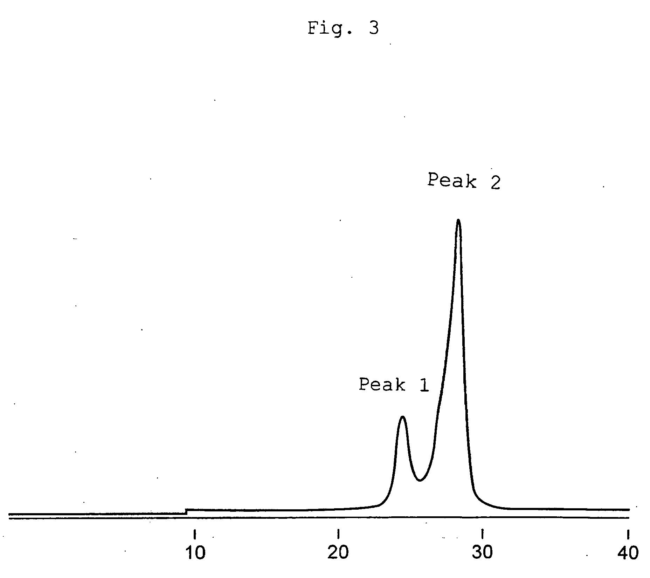

Fig. 3 shows results of gel filtration chromatography in the process of purification of selenoprotein P fragments.

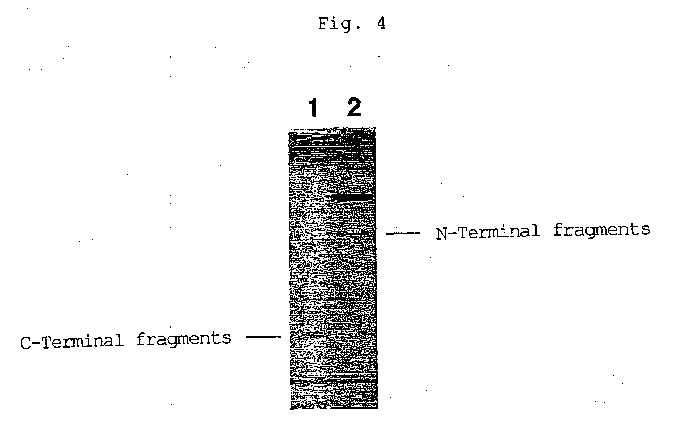

Fig. 4 shows results of SDS-PAGE electrophoresis of fractions obtained by gel filtration chromatography in the process of purification of selenoprotein P fragments. Lane 1: Peak 2 in Fig. 3; Lane 2: Peak 1 in Fig. 3.

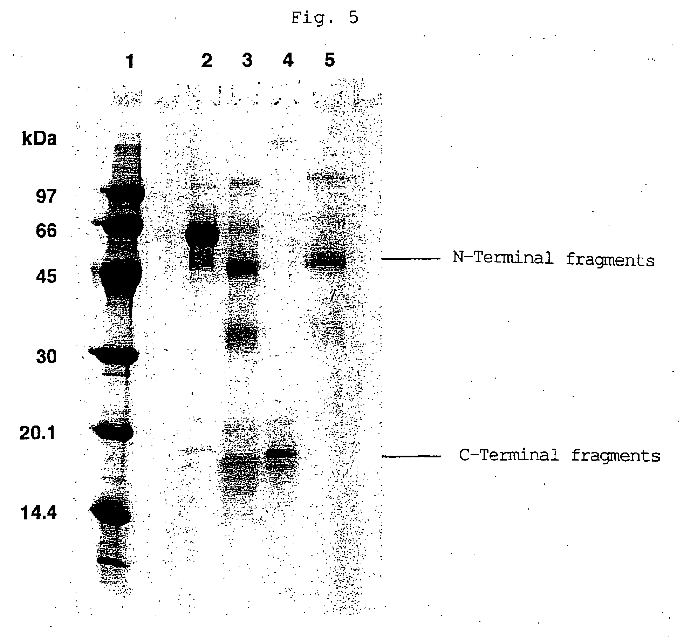

Fig. 5 shows results of SDS-PAGE electrophoresis of fractions obtained by affinity chromatography with a monoclonal antibody in the process of purification of selenoprotein P fragments. Lane 1: MW marker; Lane 2: selenoprotein P; Lane 3: a sample applied to Ni-NTA chromatography; Lane 4: an adsorbed fraction of affinity chromatography with a monoclonal antibody; Lane 5: a non-adsorbed fraction of affinity chromatography with a monoclonal antibody.

### BEST MODE FOR CARRYING OUT THE INVENTION

The process for preparing selenoprotein P fragments with the use of an enzyme as provided by the present invention firstly allows for regulation of the fragmentation process unlike the conventional processes for preparing selenoprotein P fragments that involve no specific procedure of degradation and have poor reproducibility as described above.

The thus obtained reaction products may further be used as a starting material to be applied to the conventionally used chromatographic procedures to enable purification of desired selenoprotein P fragments. The chromatographic procedures may be those conventionally used, including for instance, but not limited to, chromatography utilizing difference in a molecular weight such as gel filtration or ultrafiltration membrane partition, ion exchange chromatography, hydrophobic chromatography, reverse phase chromatography, affinity chromatography, or metal chelate chromatography, any of which may suitably be used in the present invention.

More suitably, gel filtration chromatography or affinity chromatography using as a ligand a monoclonal antibody that binds specifically to selenoprotein P fragments may be used for purifying selenoprotein P fragments with a molecular weight of not more than 3.5 x 10⁴. Metal chelate chromatography may also be used to separate desired selenoprotein P fragments from intact selenoprotein P based on the fact that the selenoprotein P fragments substantially lack the histidine rich regions. Preferably, these types of chromatography may be combined to enable more efficient purification of desired selenoprotein P fragments.

The process as described above firstly allows for stable and constant production of desired selenoprotein P fragments with a defined quality.

In order to more definitely feature the present invention, the present invention is explained in more detail by means of the following Examples which are not intended to restrict a scope of the present invention in any sense.

### Example 1

### (Purification of full-length selenoprotein P)

Purification of selenoprotein P from plasma was performed as taught by Saito Y. et al., J. Biol. Chem., vol. 274, p.2866-2871 (1999) as described below.

Human fresh lyophilized plasma (2 L) was fully thawed in a warm bath and then placed in a cooling chamber. To the plasma was added 100 g PEG 4000 in portions while stirring. After completion of addition, stirring was further continued for 1 hour. The mixture was centrifuged at 10,000 g for 20 minutes. Supernatant was recovered and filtered through AP25 (Millipore). A total of the filtrate was applied to a column charged with 100 mL heparin Sepharose (Amersham Pharmacia Biotech) previously equilibrated with 20 mM phosphate buffer (20 mM phosphate, pH 7.4, 0.15 M, 0.2 mM EDTA). After washing the column, adsorbed proteins were gradient eluted with a gradient of a salt concentration ranging from 0.15 M to 0.6 M. Each 5 mL of eluted fractions were taken, an amount of selenoprotein P contained in each fraction was determined by ELISA and fractions containing selenoprotein P were pooled. In order to avoid fragmentation of selenoprotein P during the purification procedure, DFP was added at a final concentration of 5 mM.

The fraction was then diluted 6-folds with 20 mM Tris buffer (20 mM Tris-HCl, pH 8). At this stage, a total amount of the sample was 0.75 to 1 L. The sample was applied to 40 mL Q Sepharose (Amersham Pharmacia Biotech) column equilibrated with 20 mM Tris buffer. The column was washed with 250 to 300 mL equilibration buffer and elution was performed with a gradient of a salt concentration up to 0.25 M using 250 mL buffer. The obtained factions were determined by ELISA and fractions containing selenoprotein P were pooled. To this pooled faction was added DFP at a final concentration of 5 mM.

To the obtained pooled fraction was then added imidazole at a final concentration of 2 mM and the mixture was applied to 4 mL Ni-NTA agarose gel (Qiagen) column. The column has previously been equilibrated with 20 mM Tris buffer (20 mM Tris-HCl, pH 8, 2 mM imidazole). After application, the column was washed with 30 mL washing buffer (20 mM Tris-HCl, pH 8, 20 mM imidazole, 1 M NaCl) and elution was performed with an elution buffer (20 mM Tris-HCl, pH 8, 150 mM imidazole, 1 M NaCl). Each 1 mL of eluted fractions were taken and fractions were pooled where peaked absorbance was observed at O.D. 280 nm. This fraction was concentrated with centrifugation to 2.5 mL with SpeedVac (SARVANT). Table 1 below summarizes purification yields from heparin Sepharose chromatography to Ni-NTA agarose eluate fraction.

**Table 1**

| Step | Volume (mL) | ELISA (µg/mL) | Total antigen (mg) | Yields (%) |
|---|---|---|---|---|
| Heparin Sepharose | 2000 | 2.4 | 4.8 | 100.0 |
| Q-Sepharose | 1920 | 2.7 | 5.1 | 106.3 |
| Ni-NTA agarose | 133 | 18.6 | 2.5 | 51.5 |
| Eluate | 10.5 | 227.0 | 2.4 | 49.6 |

Finally, the concentrated selenoprotein P fraction was applied to PD-10 column (Amersham Pharmacia Biotech) equilibrated with a phosphate buffer, followed by desalting and exchange of a buffer. Selenoprotein P thus obtained showed migration of M.W. around 67,000 in SDS-PAGE, most of which was thought to be full-length selenoprotein P. Selenoprotein P thus obtained was used in subsequent Examples in search of cleaving enzymes.

### Example 2

### (Enzymatic digestion of full-length selenoprotein P)

To 4 µg selenoprotein P obtained in Example 1 were added 1/10 amount of protease enzymes at a weight ratio and the mixture was reacted at 37°C for 24 hours. TBS (50 mM Tris-HCl, 0.15 M NaCl, pH 7.5) was used as a buffer in the reaction and supplemented with 5 mM CaCl₂ in case of a calcium-dependent protease. Proteases tested include serine proteinases including plasma kallikrein, factor XIIa, factor XIa, factor Xa, factor IXa, factor VIIa, α thrombin, activated protein C, plasmin, neutrophil elastase and trypsin. After the reaction was completed, samples were added with an equivalent amount of SDS solution (62.5 mM Tris-HCl, pH 6.8, 2% SDS, 0.001% Bromophenol Blue, 15% glycerol) and run on SDS-PAGE, followed by Coomassie Brilliant Blue dye. Fig. 1 shows results of SDS-PAGE electrophoresis. A non-reactive sample was prepared by adding the same amount of the enzyme and the substrate as the test samples to a reaction tube previously charged with an SDS solution.

As a result of this experiment, bands of a molecular weight of 1 x 10⁴ to not more than 3.5 x 10⁴, similar to the selenoprotein P fragments having a potent cell death-inhibitory activity (Republished publication WO00/31131 (Japanese patent application No. 347863/1998)), were observed for every proteases tested. Among the enzymes tested, plasma kallikrein, factor XIIa, factor XIa and plasmin were found to most efficiently digest selenoprotein P from comparison of an amount of the remaining intact selenoprotein P.

### Example 3

### (Cell death-inhibitory activity of selenoprotein P fragments)

In order to investigate whether the selenoprotein P fragments in the samples obtained in Example 2 have an enhanced cell death-inhibitory activity as compared to full-length selenoprotein P, an assessment system for cell death-inhibitory activity as described below was used for assessment.

To 1 ml Dami cells (Greenberg S. M. et al., Blood, vol. 72, p.1968-1977 (1988); 1 x 10⁶ cells/dish/3 ml), which can be subcultured in serum free medium SFO3 (manufactured by Sanko Jun-yaku K.K.) containing 0.05 µM β-mercaptoethanol and 0.1% BSA, was added 2 ml 1:2:2 mixed medium (SA medium) of RPMI 1640/D-MEM/F-12. The cells were cultured for three days and recovered for assay. The recovered cells were washed twice with SA/0.05% skimmed BSA (manufactured by SIGMA) containing 4 µM linoleic acid and suspended in the same medium at 3 x 10⁴ cells/mL. The cell suspension was then added to a 96-well plate in each 200 µl for wells for sample addition or in each 100 µl for wells for serial dilution. To the wells for sample addition was added 2 µl sample and, after stirring, a serial dilution was made with the wells containing 100 µl cell suspension. The plate was incubated at 37°C in CO₂ incubator for 4 to 5 days followed by assessment. In this system, Dami cells will die in the wells added with samples having no activity but will survive in the wells added with samples having the activity on Day 4 and thenceforth. Thus, assessment was made with to what folds of dilution of tested samples the living cells could survive. Therefore, the higher the value in the following table, the higher a cell death-inhibitory activity.

It was observed that the samples containing selenoprotein P fragments obtained in Example 2 exhibited at least 1.5- to 3-folds higher activity based on folds of dilution as compared to full-length selenoprotein P. This proved that selenoprotein P fragments with potent cell death-inhibitory activity could actually be prepared by using the enzymes as described above in accordance with the present invention.

**Table 2**

| Enzymes used for cleavage | Cell death-inhibitory activity (Folds of dilution) |
|---|---|
| Selenoprotein P untreated | 400 |
| Plasma kallikrein | 1200 |
| Factor XIIa | 600 |
| Factor XIa | 800 |
| Factor Xa | 800 |
| Factor IXa | 600 |
| Thrombin | 600 |
| Plasmin | 600 |
| Activated protein C | 800 |
| Elastase | 600 |
| Trypsin | 600 |

### Example 4

### (Digestion of full-length selenoprotein P with kallikrein and plasmin)

In practically preparing selenoprotein P fragments, an amount of an enzyme to be reacted with selenoprotein P or a reaction time may depend on efficiency of each enzyme to the enzyme substrate. Thus, efficiency of an enzymatic digestion of selenoprotein P was characterized for plasma kallikrein and plasmin by observing enzymatic digestion in course of time. An amount of the enzyme to be added was the same as described in Example 2. Samples were taken 0, 2, 4, 6, 8, 10, 12 and 24 hours after initiation of the reaction for plasma kallikrein and 0, 5, 10, 15, 20, 25, 30 and 60 minutes after initiation of the reaction for plasmin, and run on SDS-PAGE to observe progress of cleavage. Fig. 2 shows the results of SDS-PAGE electrophoresis. As a result, it was observed for either of these enzymes that selenoprotein P was continually cleaved in course of time to produce selenoprotein P fragments corresponding to a molecular weight of about 2 x 10⁴.

It was thus proved that desired selenoprotein P fragments could be prepared by suitably adjusting the reaction conditions depending on a kind of enzymes used for preparation of fragments.

### Example 5

### (Process for purification of selenoprotein P fragments)

Selenoprotein P fragments produced by restricted enzymatic degradation may be purified by the procedures described below. Selenoprotein P fragments with a potent cell death-inhibitory activity (C-terminal molecules of selenoprotein P) lack an ability to bind to metal chelate as they substantially lack the histidine rich regions of selenoprotein P. Using this property, C-terminal molecules of selenoprotein P may be prepared.

To 1 mg selenoprotein P was added 50 µg plasma kallikrein and the mixture was reacted at 37°C for 24 hours so that selenoprotein P is restrictedly degraded. Upon completion of the reaction, DFP was added at a final concentration of 1 mM to thereby inactivate kallikrein. Thereto was added imidazole at a final concentration of 2 mM and the mixture was applied to 3 mL Ni-NTA agarose gel previously equilibrated with an equilibration buffer (20 mM Tris-HCl, 2 mM imidazole, pH 8). The column was washed with 30 mL washing buffer (20 mM Tris-HCl, 20 mM imidazole, 1 M NaCl, pH 8) and these non-adsorbed fractions and washing fractions were recovered. The C-terminal molecules of selenoprotein P that were produced after restricted degradation with kallikrein do not bind to Ni-NTA agarose gel as they substantially lack the histidine rich regions. Hence, the C-terminal molecules of interest may be recovered in these non-adsorbed fractions and washing fractions of chromatography. N-terminal molecules may also be recovered from this column using an elution buffer (20 mM Tris-HCl, 120 mM imidazole, 1 M NaCl, pH 8).

For these non-adsorbed fractions and washing fractions, concentration and buffer exchange was performed by repeating concentration with CentriFlow and dilution with PBS. The fractions were finally concentrated up to 1 mL and applied to Superose 12HR10/30 column, equilibrated with PBS, for gel filtration with PBS as a buffer at a flow rate of 0.5 mL/min to monitor at a wave length 220 nm. As a result, two main peaks were detected, the first peak representing N-terminal fragment of selenoprotein P and intact full-length selenoprotein P that remained without cleavage, and the second peak representing C-terminal molecule (cf. Fig. 3). Fig. 4 shows results of SDS-PAGE electrophoresis of the peak fractions obtained in the process of gel filtration as described above.

### Example 6

### (Process for purification of selenoprotein P fragments)

Affinity chromatography with a monoclonal antibody is also suited for purifying more specifically and efficiently C-terminal fragments of selenoprotein P. A monoclonal antibody for this purpose may preferably be those recognizing C-terminal molecule of selenoprotein P as an antigen, more preferably a monoclonal antibody obtained after immunization of mice with the selenoprotein P fragments having a potent cell death-inhibitory activity. Such a monoclonal antibody may be bound e.g. to Sepharose gel activated with cyanogen bromide to prepare monoclonal antibody-bound gel. With a column chromatography using such gel, C-terminal fragments of selenoprotein P may directly be purified from the solution of restricted degradation of selenoprotein P with an enzyme, or from the non-adsorbed fractions and washing fractions of Ni-NTA agarose gel as prepared in Example 5.

The non-adsorbed fractions and washing fractions of Ni-NTA agarose gel obtained under the same conditions as in Example 5 were directly applied to 1.5 mL monoclonal antibody affinity column equilibrated with PBS (20 mM Na₂HPO₄-NaH₂PO₄, 0.15 M NaCl, pH 7.4). After washing the column with about 8 mL PBS, elution was made with 0.1 M glycine, pH 2.8. The eluate fractions were neutralized with about 1/10 amount of 1 M Tris-HCl. The fraction that passed through chromatographic column and the washing fractions were pooled, concentrated with SpeedVac, and dialyzed against PBS. As for the eluate fractions, those fractions exhibiting absorbance at 220 nm were pooled, and similarly concentrated and dialyzed. As a result of electrophoresis, N-terminal fragment of selenoprotein P from the fraction that passed through chromatographic column and the washing fractions, and C-terminal fragment of selenoprotein P from the eluate fractions, were detected, respectively (cf. Fig. 5). For the purpose of assessment as to a cell death-inhibitory activity as described in Example 3, a cell death-inhibitory activity was determined for the samples by measuring a content of selenium in each of the samples with atomic absorption spectrometer and adding each 1 ppm selenium to the system.

**Table 3**

| Sample (Each 1 ppm selenium) | Cell death-inhibitory activity (Folds of dilution) |
|---|---|
| Inorganic selenium | 5000 |
| Full-length selenoprotein P | 5000 |
| After enzymatic digestion | 6000 |
| Purified N-terminal fragment | 1800 |
| Purified C-terminal fragment | 12000 |

It was observed that C-terminal fragment of selenoprotein P exhibited several times higher cell death-inhibitory activity per one selenium atom than full-length selenoprotein P.

## Claims

1. A process for preparing a peptide fragment having a cell death-inhibitory activity which comprises reacting selenoprotein P or a fraction containing said protein with a serine proteinase.

2. The process according to claim 1 wherein said serine proteinase is selected from the group consisting of plasma kallikrein, factor XIIa, factor XIa, factor Xa, factor IXa, factor VIIa, thrombin, plasmin, tissue plasminogen activator, urokinase, trypsin, and neutrophil elastase.

3. The process according to claim 2 wherein said serine proteinase is plasma kallikrein, factor XIIa, factor XIa or plasmin.

4. The process according to any one of claims 1 to 3 which comprises adding not more than 1/10 amount of a serine proteinase at a weight ratio to selenoprotein P, and incubating the mixture at 37°C for 15 minutes to 24 hours.

5. A use of a serine proteinase selected from the group consisting of plasma kallikrein, factor XIIa, factor XIa, factor Xa, factor IXa, factor VIIa, thrombin, plasmin, tissue plasminogen activator, urokinase, trypsin, and neutrophil elastase for producing a peptide fragment having a cell death-inhibitory activity from selenoprotein P.

6. The use according to claim 5 wherein said serine proteinase is plasma kallikrein, factor XIIa, factor XIa or plasmin.

7. A process for purifying a peptide fragment having a cell death-inhibitory activity which comprises reacting selenoprotein P or a fraction containing selenoprotein P with a serine proteinase to prepare a solution containing selenoprotein P fragments, and subjecting said solution containing selenoprotein P fragments to gel filtration chromatography, affinity chromatography with a monoclonal antibody to selenoprotein P fragment as a ligand, and/or metal chelate chromatography.
